# EUROPÄISCHE PATENTSCHRIFT

(11) **EP 3 692 938 B1**
(45) Veröffentlichungstag und Bekanntmachung des Hinweises auf die Patenterteilung: **20.05.2026**
(21) Anmeldenummer: 20154609.0
(22) Anmeldetag: 30.01.2020
(51) Int. Cl.: A61B 18/14

(54) **LÖSBARER ISOLIEREINSATZ ZUR VERWENDUNG IN EINEM RESEKTOSKOP**
RELEASABLE INSULATING INSERT FOR USE IN RESECTOSCOPE
INSERT ISOLANT DÉTACHABLE DESTINÉ À ÊTRE UTILISÉ DANS UN RÉSECTOSCOPE

(30) Priorität: 05.02.2019 DE 102019102841
(43) Veröffentlichungstag der Anmeldung: 12.08.2020
(73) Patentinhaber: Olympus Winter & Ibe GmbH, 22045 Hamburg (DE)
(72) Erfinder: BROCKMANN, Christian, 21279 Hollenstedt (DE); Offt, Andreas, 21465 Reinbek (DE)
(74) Vertreter: Hoener, Matthias

(56) Entgegenhaltungen:
- DE-A1- 102013 001 156
- DE-C1- 10 122 465
- DE-C5- 10 122 465
- US-A1- 2002 188 293
- US-A1- 2005 080 412

## Beschreibung

### Hintergrund

Die Erfindung betrifft einen elektrisch isolierenden Isoliereinsatz der im Oberbegriff des Anspruchs 1 genannten Art sowie ein Elektrodeninstrument und ein Resektoskop der im Oberbegriff der Ansprüche 6 bzw. 8 genannten Art.

Resektoskope der gattungsgemäßen Art, die entsprechende Elektrodeninstrumente und an ihrem distalen Ende eine Isolierspitze aufweisen, werden vor allem in der Urologie bei elektrochirurgischen Arbeiten in der Blase und der Urethra verwendet. Sie werden üblicherweise zur Resektion und Vaporisation von Gewebe, zum Beispiel von Gewebe im unteren Harntrakt, verwendet. Dazu umfassen die Resektoskope ein längsverschiebbares elektrochirurgisches Durchgangsinstrument, das nach dem Einführen des Resektoskops mit seinem distalen Arbeitsende aus dem distalen Ende des Schaftrohres des Resektoskops hervorgeschoben werden kann. Das elektrochirurgische Durchgangsinstrument kann an seinem distalen Arbeitsende eine elektrochirurgische Elektrode, zum Beispiel in Form eines Loops (Schlinge) oder Vaporisationsknopfes (z. B. PlasmaButtons), umfassen. Solche Instrumente sind zum Beispiel die OES PRO Resektoskope (Olympus) oder andere Dauerspül-Resektoskope nach Iglesias.

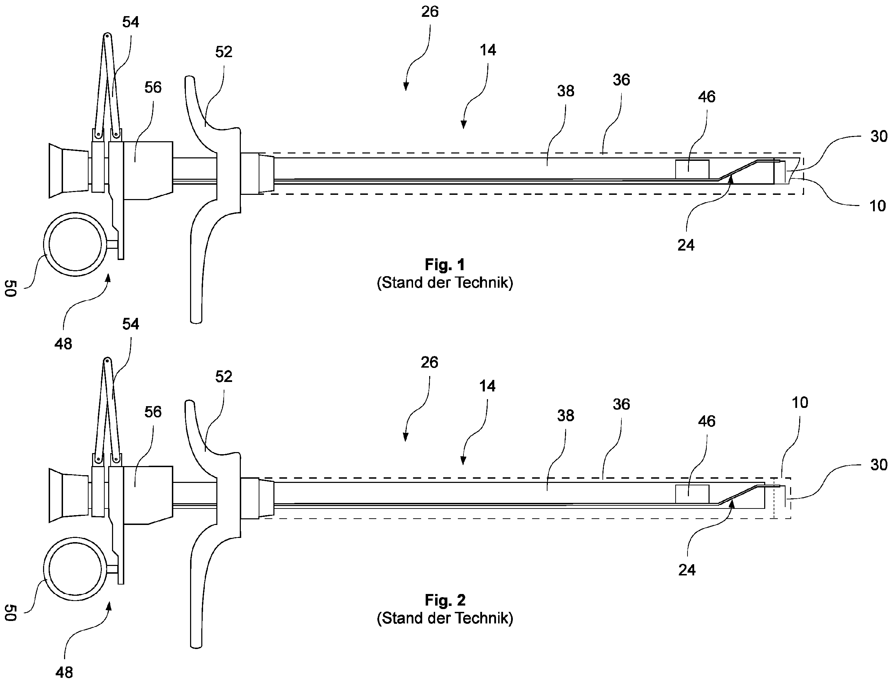

Um Kurzschlüsse zwischen der Aktivelektrode und dem leitfähigen Material des Schaftrohres zu vermeiden, umfassen die Resektoskope in ihrem distalen Endbereich üblicherweise einen Abschnitt, der aus einem isolierenden Material, wie einer Keramik, gefertigt ist und üblicherweise als Isolierspitze oder Isoliereinsatz bezeichnet wird. Der Isoliereinsatz kann, wie in Fig. 1 bzw. 2 gezeigt, entweder am Innenschaft (Fig. 1) oder am Außenschaft (Fig. 2) angeordnet sein. Da das Resektoskop auf Mehrfachverwendung ausgelegt ist und entsprechend auch Sterilisierungsbedigungen, z.B. Autoklavieren, wiederstehen muss, muss der Isoliereinsatz in beiden Fällen hohe Anforderungen an Haltbarkeit und Wiederaufbereitbarkeit erfüllen. Die Werkstoffauswahl ist daher in der Regel auf relativ teure Hochleistungskeramiken wie Siliziumnitrit beschränkt. Um die Zerbrechlichkeit der aus Keramik gefertigten Isoliereinsätze auf ein Minimum zu reduzieren, müssen die Einsätze darüber hinaus eine relativ hohe Wandstärke aufweisen.

Relevanter Stand der Technik ist in den Dokumenten US 2005/080412 A1, DE 10 2013 001156 A1, DE 101 22 465 C1 und US 2002/188293 A1 offenbart.

Es wäre wünschenswert, sowohl die Kosten für die Isoliereinsätze zu reduzieren als auch deren Wandstärken weiter verringern zu können. Es besteht daher ein Bedarf an entsprechend verbesserten Isoliereinsätzen.

### Beschreibung

Gelöst wird diese Aufgabe durch einen Isoliereinsatz mit den Merkmalen von Anspruch 1, ein Elektrodeninstrument mit den Merkmalen von Anspruch 6 und ein Resektoskop mit den Merkmalen von Anspruch 8.

Erfindungsgemäß wird der Isoliereinsatz insbesondere als Teil zur einmaligen Verwendung ausgebildet, das lösbar mit dem Resektoskopschaft verbunden ist und somit nach erfolgtem Einsatz auf einfache Weise ausgetauscht und durch einen neuen Isoliereinsatz ersetzt werden kann. Gleichzeitig ist der Isoliereinsatz mittels eines Befestigungsmittels sicher an dem Resektoskopschaft gehalten, sodass ein Verlust des Isoliereinsatzes während eines Eingriffs ausgeschlossen ist.

In einem ersten Aspekt betrifft die Erfindung daher einen elektrisch isolierenden Isoliereinsatz zur lösbaren Verbindung mit dem distalen Endbereich eines Resektoskopschaftes, dadurch gekennzeichnet, dass der Isoliereinsatz einen hohlen Abschnitt mit einem länglichen Hohlraum zum Hindurchführen von Durchgangsinstrumenten aufweist und dass der Isoliereinsatz Befestigungsmittel zur lösbaren Verbindung mit dem Resektoskopschaft aufweist.

Der Isoliereinsatz ist elektrisch nicht leitfähig, d.h. elektrisch isolierend, ausgebildet. Auf diese Weise wird die Isolierung der Aktivelektrode gegenüber dem leitfähigen Resektoskopschaft gewährleistet. Zu diesem Zweck ist der Isoliereinsatz vorzugsweise vollständig, mindestens aber in einem Maße, das die Isolierfähigkeit des Einsatzes gewährleistet, aus einem elektrisch nicht leitfähigen, d.h. elektrisch isolierenden, Material ausgebildet. Solche Materialien sind Fachleuten bekannt und umfassen beispielsweise Keramiken und Kunststoffe. Isoliereinsätze aus Kunststoffen sind erfindungsgemäß aufgrund der relativ geringen Produktionskosten und guten Isolierfähigkeit besonders bevorzugt. Da der Isoliereinsatz bei Verwendung während einer elektrochirurgischen Behandlung mit einer Elektrode in Kontakt mit dem entstehenden Plasma kommen kann, sind thermostabile Kunststoffe besonders bevorzugt. Thermostabile Kunststoffe sind in der Lage, die im Bereich der distalen Resektoskopspitze entstehenden hohen Temperaturen unbeschadet zu überstehen. Geeignete thermostabile Kunststoffe können beispielsweise ausgewählt sein aus der Gruppe bestehend aus Fluorpolymeren und Cycloolefin-Copolymeren. Die aus Kunststoff gefertigten Isoliereinsätze können mittels eines Spritzgussverfahrens gefertigt sein.

Der Isoliereinsatz ist zur lösbaren Verbindung mit dem distalen Endbereich eines Resektoskopschaftes geeignet. Dies bedeutet, dass Isoliereinsatz und der Endbereich mindestens abschnittsweise form- und größenkomplementär sind. Hierdurch ist der Isoliereinsatz mit dem Endbereich verbindbar. Verschiedene mögliche Ausführungsformen für diese Verbindung sind an anderer Stelle hierin beschrieben. Jedenfalls ist die Verbindung derart fest bzw. derart gesichert, dass ein Ablösen des Isoliereinsatzes während eines operativen Eingriffs verhindert ist.

Gleichzeitig ist die Verbindung zwischen dem Resektoskopschaft und dem Isoliereinsatz lösbar ausgebildet, um einen leichten Austausch und/oder Reinigung des Isoliereinsatzes zu ermöglichen, zum Beispiel durch das medizinische Fachpersonal oder das für die Wiederaufbereitung des Instruments zuständige Reinigungsfachpersonal, d.h. den Endnutzern des Resektoskops. So ist der Isoliereinsatz insbesondere nicht mit dem Endbereich des Resektoskopschafts verklebt. Dies schließt jedoch nicht aus, dass die Lösung der Verbindung zwischen dem Isoliereinsatz und dem Resektoskopschaft, zur Reinigung übliche Demontageschritte voraussetzen kann. So ist es in bestimmten Ausführungsformen der Erfindung beispielsweise erforderlich, den Außenschaft (Hüllrohr) von dem Innenschaft mit eingeführtem Elektrodeninstrument zu lösen, bevor der Isoliereinsatz von dem Innenschaft oder dem Außenschaft gelöst werden kann.

Die Verbindung zwischen dem Resektoskopschaft und dem Isoliereinsatz wird in dem distalen Endbereich des Schaftes hergestellt. In der Regel wird der Isoliereinsatz hierzu mindestens abschnittsweise formkomplementär zu Elementen des Resektoskopschaftes ausgebildet sein, die in dessen Endbereich angeordnet sind. So kann der Isoliereinsatz, der einen zylindrischen Abschnitt aufweist, beispielsweise auf das Innen- oder Außenrohr aufgeschoben, in das Innenrohr eingeschoben oder zwischen Innen- und Außenrohr eingeschoben werden. Dabei ist die letztgenannte Möglichkeit bevorzugt. Der Isoliereinsatz kann beispielsweise mit dem Innenrohr verbunden und so ausgeformt sein, dass das mit dem Isoliereinsatz verbundene Innenrohr aus proximaler Richtung in ein Außenrohr eingeführt werden kann.

Im montierten Zustand überlappen sich der Isoliereinsatz und der Resektoskopschaft daher in der Regel, d.h. sie greifen ineinander ein. Es versteht sich, dass der Isoliereinsatz dabei nicht vollständig in den Resektoskopschaft eingeführt sein sollte, um eine ausreichende Isolierung der Elektrode gewährleisten zu können. Der sich überlappende Bereich und damit der hier erwähnte "distale Endbereich" des Resektoskopschaftes wird sich daher ausgehend von dem distalen Ende des Resektoskopschaftes in der Regel auf einen distalen Abschnitt von wenigen mm beschränken, z.B. 10 mm oder weniger, 8 mm oder weniger, bevorzugt 5 mm oder weniger.

Wie erwähnt weist der Isoliereinsatz einen hohlen Abschnitt mit einem länglichen Hohlraum zum Hindurchführen von Durchgangsinstrumenten auf. Dieser Abschnitt ist im proximalen Endbereich des Isoliereinsatzes angeordnet. Er stellt sicher, dass durch den Resektoskopschaft geführte Durchgangsinstrumente durch das kanalförmige Innere des Isoliereinsatzes hindurchgeführt werden können. Der Isoliereinsatz kann eine im Wesentlichen zylindrische Form aufweisen oder mindestens einen im Wesentlichen zylindrischen proximalen Abschnitt. Entsprechend kann der Hohlraum im Inneren des hohlen Abschnitts eine Hohlzylinderform aufweisen. Im Rahmen der Erfindung ist es aber auch angedacht, den Hohlraum und den entsprechenden Abschnitt mit nichtkreisförmigen Querschnitten auszubilden. So kann es für bestimmte Anwendungen vorteilhaft sein, den Hohlraum und/oder die Außenseite des Abschnitts mit einem elliptischen oder ovalen Querschnitt auszubilden. Auch unregelmäßige Querschnitte sind denkbar, z.B. Einbuchtungen oder Ausstülpungen in der Innenwand des Abschnitts zur Führung bestimmter Durchgangsinstrumente. Drüber hinaus ist es angedacht, die Wandstärke des Abschnitts an ihre jeweilige Belastung anzupassen. So kann beispielsweise die Wand in einem nach distal verlängerten Bereich dicker ausgebildet werden, als die Wand in anderen Bereichen des Isoliereinsatzes. Insgesamt kann der hohle Abschnitt als rohrförmig angesehen werden, wobei der Begriff "Rohr", wie zuvor beschrieben, nicht nur reine Hohlzylinderformen einschließt, sondern längliche Abschnitte mit einer Außenwand und einem länglichen Hohlraum in ihrem Inneren, wobei der Hohlraum an seinem distalen und proximalen Ende offen ist, um das Hindurchführen von Durchgangsinstrumenten zu ermöglichen. Hindurchzuführende "Durchgangsinstrumente" können beispielsweise Elektrodeninstrumente, Optiken, Spülrohre und dergleichen umfassen.

Im Übrigen kann der Isoliereinsatz, insbesondere in seinem distalen Endbereich, übliche Formen aufweisen. Dabei sind die Wände des Isoliereinsatzes bevorzugt in dem Wandraum eines hohlzylindrischen Raumes angeordnet. Unterschiedliche Wanddicken sind jedoch, wie oben angedeutet, auch im Rahmen der Erfindung erlaubt. Bevorzugt weist der Wandraum den gleichen Innendurchmesser wie ein im Resektoskopschaft angeordnetes Innenrohr auf. In der Vergangenheit hat sich insbesondere die in Fig. 1 gezeigte, einseitig nach distal verlängerte Form für Isolierspitzen bewährt. Der nach distal verlängerte Bereich sowie der proximal davon angeordnete Bereich des hohlen Abschnitts können eine größere Wanddicke aufweisen als andere Bereiche des Isoliereinsatzes. Mit derart gestalteten, "schnabelförmigen" Isoliereinsätzen kann Gewebe an der verlängerten Seite des Einsatzes abgestützt werden, so dass das Gewebe nicht in den Sichtbereich einfallen kann. Darüber hinaus wird durch diese Gestaltung der Weg der Spülflüssigkeit verlängert und damit ein gradliniger Strom erzeugt, der die Gefahr von Verwirbelungen vor der Optik reduziert. Gleichzeitig kann die Elektrode weiterhin nah an der Kante des Isoliereinsatzes Gewebe durchtrennen.

Erfindungsgemäß weist der Isoliereinsatz ein oder mehrere Befestigungsmittel zur lösbaren Verbindung mit dem Resektoskopschaft auf. Das oder die Befestigungsmittel kann verschiedene Formen annehmen und stellt in Verbindung mit der Ausbildung des distalen Endbereichs des Resektoskopschaftes eine sichere, aber lösbare Verbindung sicher. Die Befestigungsmittel sind in der Regel in dem proximalen Endbereich des Isoliereinsatzes angeordnet, vorzugsweise in dessen hohlen Abschnitt.

Das Befestigungsmittel kann beispielsweise eine radiale Verdickung des Isoliereinsatzes in dessen hohlen Abschnitt umfassen oder aus dieser bestehen. Die Verdickung kann in Eingriff mit einem weiteren Element an dem Resektoskopschaft stehen und auf diese Weise den Isoliereinsatz gegen eine Verschiebung in distale Richtung sichern. Das weitere Element an dem Resektoskopschaft kann beispielsweise eine Eingriffsöffnung oder eine Protrusion sein. Die Verdickung kann flexibel, beispielsweise als Schnappelement, ausgebildet sein, um ein Aufschieben des Isoliereinsatzes auf den Resektoskopschaft zu ermöglichen. Alternativ kann die Verdickung unflexibel sein, wenn beispielsweise der Isoliereinsatz bei der Montage des Resektoskops zwischen Strukturen des Innenrohrs und des Außenrohrs angeordnet wird.

Die radiale Verdickung kann beispielsweise in a) einem größeren Außendurchmesser des Isoliereinsatzes im Verhältnis zu dem Außendurchmesser am distalen Ende des hohlen Abschnitts bestehen, und/oder b) einem größeren Durchmesser der Wand des Isoliereinsatzes im Verhältnis zu dem Durchmesser der Wand am distalen Ende des hohlen Abschnitts bestehen. In einem Beispiel eines größeren Außendurchmessers gemäß Alternative a) kann der Isoliereinsatz, insbesondere dessen Außenwand, vollständig oder teilweise in axialer Richtung konisch ausgebildet sein. Der Außendurchmesser des Isoliereinsatzes an dessen proximalen Ende wäre dann größer als dessen Außendurchmesser an dessen distalen Ende. In einem Beispiel eines größeren Durchmessers der Wand des Isoliereinsatzes gemäß Alternative b) können auf der Außen- oder Innenwand des Isoliereinsatzes eine oder mehrere Protrusionen, zum Beispiel in Form von Zapfen, ausgebildet sein. Die Protrusion kann radial umlaufend auf der entsprechenden Wand ausgebildet sein oder als ein oder mehrere Zapfen, die radial entlang eines Umfangs der entsprechenden Wand angeordnet sind.

Alternativ oder zusätzlich kann das Befestigungsmittel einen Teil einer in anderen Bereichen üblichen Verbindung umfassen oder aus diesem bestehen. So kann das Befestigungsmittel beispielsweise einen Teil einer Bajonett-, Schraub-, Schnapp-, Klemm- oder Rastverbindung umfassen oder aus dieser bestehen. Das oder die entsprechende komplementäre Teil(e) der jeweiligen Verbindung wird dann an dem Resektoskopschaft ausgebildet sein, so dass zwischen dem Isoliereinsatz und dem Resektoskopschaft eine Bajonett-, Schraub-, Schnapp-, Klemm- bzw. Rastverbindung ausgebildet ist.

In einer Ausführungsform umfasst das Befestigungsmittel beispielsweise ein an der Befestigungsseite des hohlen Abschnitts angeordnetes, vorstehendes Rastelement umfasst. Als "Befestigungsseite" wird hierin diejenige von Außenseite und Innenseite des hohlen Abschnitts des Isoliereinsatzes angesehen, an der das Befestigungsmittel angeordnet ist. Bevorzugt ist die Befestigungsseite des hohlen Abschnitts dessen Außenwand. Dies bedeutet beispielsweise, dass bei einem zwischen Außenrohr und Innenrohr des Resektoskopschafts angeordneten Bereich des Isoliereinsatzes, das Außenrohr Elemente zur Absicherung des Isoliereinsatzes gegen eine distale Verschiebung aufweist.

Während der Isoliereinsatz gegen eine axiale Verschiebung gesichert ist, kann er in bestimmten Ausführungsformen rotierbar an dem Innen- oder Außenrohr bzw. zwischen Innen- und Außenrohr angeordnet sein. Dies ermöglicht eine Rotation des Isoliereinsatzes bei einem Eingriff, sodass Gewebe aus verschiedenen Richtungen erreichbar ist. Die Rotation des Isoliereinsatzes kann mit der Rotation der Elektrode um die Längsachse des Resektoskopschaftes synchronisiert bzw. an diese gekoppelt sein.

In einem verwandten, zweiten Aspekt betrifft die Erfindung ein Elektrodeninstrument zur Verwendung in einem Resektoskop, wobei das Elektrodeninstrument einen Schaftabschnitt und an seinem distalen Ende eine mit Hochfrequenzstrom beaufschlagbare Elektrode aufweist, dadurch gekennzeichnet, dass das Elektrodeninstrument mit einem erfindungsgemäßen Isoliereinsatz verbunden ist und das Elektrodeninstrument und der Isoliereinsatz im Verhältnis zueinander axial verschiebbar sind.

Die in Resektoskopen verwendeten Elektrodeninstrumente sind oftmals für die einmalige Verwendung (single use) ausgebildet. Auch der erfindungsgemäße Isoliereinsatz kann für die einmalige Verwendung ausgebildet sein. Die vorgenannte Verbindung zwischen Elektrodeninstrument und Isoliereinsatz ermöglicht es vorteilhafterweise zwei potentiell für die einmalige Verwendung ausgelegte Komponenten des Systems gemeinsam zum Austausch anzubieten. Darüber hinaus kann der Isoliereinsatz auf diese Weise auch bei der Führung und Positionierung der Elektrode im Schaftsystem mitwirken. Gleichzeitig kann die Schlinge der Elektrode genauer zum Isoliereinsatz ausgerichtet werden. Der Isoliereinsatz kann zusätzlich bei dem Einführen des mit dem Isoliereinsatz verbundenen Elektrodeninstruments als Griffkörper verwendet werden, d.h. als Körper an dem die verbundenen Teile greifbar sind. Dadurch wird das Elektrodeninstrument, insbesondere die Elektrode beim Einführen geschont und die Handhabbarkeit für den Endnutzer verbessert.

Das Elektrodeninstrument weist einen langgestreckten Schaftabschnitt (Schaftteil) auf und ist als Durchgangsinstrument für ein Resektoskop ausgebildet, d. h. als Instrument, das durch ein resektoskopisches Schaftrohr in eine Körperöffnung einführbar ist. Das Elektrodeninstrument weist an seinem distalen Ende eine mit Hochfrequenzstrom beaufschlagbare Elektrode auf. Bei der Elektrode kann es sich um eine Schneidschlinge, einen PlasmaButton oder andere handelsübliche Elektroden handeln. Bevorzugt ist die Elektrode eine Schneidschlingenelektrode. Entsprechende Elektroden und Elektrodeninstrumente sind Fachleuten bekannt.

Das Elektrodeninstrument kann ein bipolares Elektrodeninstrument sein, das die Elektrode als Teil einer Elektrodenanordnung umfasst. In diesem Falle wird das Elektrodeninstrument zum Beispiel eine zweite Elektrode im distalen Endbereich des Elektrodeninstruments umfassen, die als Neutralelektrode ausgebildet ist. Alternativ kann die zweite Elektrode (Neutralelektrode) auch an anderen Elementen des distalen Endbereichs des Resektoskops angeordnet sein. Selbstverständlich kann das Elektrodeninstrument auch als monopolares Instrument ausgebildet sein.

Das Elektrodeninstrument ist innerhalb des Schaftes eines Resektoskops längsverschiebbar, d. h. in axialer Richtung nach distal und proximal beweglich. Zum Anschluss an das Resektoskop weist das Elektrodeninstrument mindestens einen langgestreckten Schaft auf, der zur Herstellung einer bewegungsgekoppelten Verbindung an seinem proximalen Ende an einem von dem Resektoskop umfassten Schlitten befestigbar ist. Der Schlitten gleitet typischerweise auf einem Rohr und wird über eine Federeinheit federvorgespannt in einer Ruhestellung gehalten. So kann die Elektrode am distalen Ende auf zu schneidendes Gewebe zu- oder von diesem wegbewegt werden, ohne das gesamte Resektoskop bewegen zu müssen. Darüber hinaus ist es durch die Längsverschiebbarkeit des Elektrodeninstruments möglich, zwischen der Elektrode und dem Isoliereinsatz Gewebe einzuklemmen und von der Eingriffsstelle zu entfernen. Das distale Ende des Isoliereinsatzes und die Elektrode sind somit mittels der Längsverschiebbarkeit des Elektrodeninstruments aufeinander zu und voneinander weg beweglich.

Erfindungsgemäß ist insbesondere ein Elektrodeninstrument, das mit einem erfindungsgemäßen Isoliereinsatz verbunden ist. Die axiale Längsverschiebbarkeit von Elektrodeninstrument und Isoliereinsatz im Verhältnis zueinander wird durch diese Verbindung nicht verhindert. Stattdessen gewährleistet die Verbindung eine Längsverschiebbarkeit um eine bestimmte Distanz in axialer Richtung. Die Distanz umfasst die Distanz um die das Elektrodeninstrument üblicherweise in axialer Richtung bewegbar ist. Gleichzeitig wird die Bewegbarkeit in andere Richtungen durch die Verbindung reduziert oder verhindert.

Der Isoliereinsatz kann beispielsweise ein oder mehr, vorzugsweise zwei Verbindungselemente aufweisen, in denen jeweils ein Gabelrohr des Elektrodeninstruments axial verschiebbar gelagert ist. Das Verbindungselement kann an der Innenwand des Isoliereinsatzes angeordnet sein. Um den Materialaufwand zu reduzieren und die Passform zu optimieren, können das oder die Verbindungselemente beispielsweise als Spritzgussteil mit dem Isoliereinsatz einteilig ausgebildet sein. Da die Elektrodeninstrumente vorzugsweise zwei Gabelrohre aufweisen, ist es entsprechend bevorzugt, dass der Isoliereinsatz zwei Verbindungselemente aufweist, in denen diese Gabelrohre jeweils gelagert werden können. Die Längsachse der Verbindungselemente erstreckt sich somit jeweils parallel zur Längsachse des Isoliereinsatzes. Die Verbindungselemente sind entsprechend über ihre Außenwand mit der Innenwand des Isoliereinsatzes verbunden. Die Verbindungselemente können rohrförmig sein, d.h. eine hohlzylindrische Form aufweisen. Alternativ zu diesen vollrohrförmigen Verbindungselementen, ist auch die Verwendung von teilzylinderförmigen Verbindungselementen im Rahmen der Erfindung möglich, d.h. Verbindungselementen mit einem teilkreisförmigen Querschnitt. Die Verbindungselemente können beispielsweise als Clips bzw. Klemmen ausgebildet sein.

In einem weiteren verwandten Aspekt betrifft die Erfindung ein Resektoskop für die endoskopische Chirurgie mit einem rohrartigen Resektoskopschaft, der ein langgestrecktes Hüllrohr und ein in dem Hüllrohr angeordnetes Innenrohr sowie eine stabförmige Optik umfasst, dadurch gekennzeichnet, dass das Resektoskop
a) einen erfindungsgemäßen Isoliereinsatz sowie ein Elektrodeninstrument mit einem Schaftabschnitt und mit einer mit Hochfrequenzstrom beaufschlagbaren Elektrode an seinem distalen Ende aufweist; oder
b) ein erfindungsgemäßes Elektrodeninstrument aufweist.

Die erfindungsgemäßen Resektoskope können in allen Bereichen der endoskopischen Chirurgie eingesetzt werden. Sie sind besonders gut für die Verwendung in engen Körperkanälen, wie der Urethra geeignet. Zu diesem Zweck weisen die Resektoskope den bereits beschriebenen rohrartigen Resektoskopschaft auf. Der Schaft kann in üblicher Weise ein Hüllrohr (Außenrohr) und ein durch das Hüllrohr hindurch verlaufendes langgestrecktes Innenrohr aufweisen, wobei das erfindungsgemäß verwendete Elektrodeninstrument vorzugsweise im Innenrohr angeordnet ist. Die Wand des erfindungsgemäßen Isoliereinsatzes in dessen proximalen Endbereich ist dabei, wie vorstehend bereits angedeutet, vorzugsweise zwischen dem Hüllrohr und dem innerhalb des Hüllrohres verlaufenden Innenrohr angeordnet.

Zur Betrachtung des Eingriffsgebietes und Überwachung des Eingriffes weist das erfindungsgemäße Resektoskop ferner eine Optik, d. h. einen optischen Bildleiter, auf. Die Optik durchläuft den Schaft über seine Länge und ist beispielsweise ebenfalls in dem Innenrohr angeordnet. Die Optik kann ein geordnetes Faserbündel umfassen und/oder hintereinander angeordnete Stablinsen. Am distalen Ende weist die Optik ein Objektiv auf und am proximalen Ende ein Okular. Das Auge des Betrachters betrachtet durch die Optik ein Beobachtungsgebiet, das vor der distalen Stirnfläche des Schaftes liegt. Alternativ kann die Optik an ihrem proximalen Ende auch mit einer digitalen Bildaufnahmeeinheit, verbunden sein.

Es ist denkbar, einzelne Komponenten, die den Schaftteil des Resektoskops durchlaufen, gegeneinander zu stabilisieren, insbesondere gegen eine Verschiebung in radiale Richtung.

So weist das Elektrodeninstrument üblicherweise Führungselemente auf, die zur Abstützung und Stabilisierung des Elektrodeninstruments innerhalb des Innenrohres dienen. Zu diesem Zweck grenzen die Führungselemente an die Innenwandung des Innenrohres oder der Optik derart an, dass eine Bewegung des Elektrodeninstruments in axialer Richtung und potentiell auch Drehbewegungen um die Längsachse möglich sind, während Bewegungen des Elektrodeninstruments in radiale Richtung reduziert oder verhindert werden. Es hat sich herausgestellt, dass es besonders vorteilhaft ist, die Führungselemente teilweise formkomplementär zur Innenwandung auszubilden. Die Führungselemente können beispielsweise einen teilkreisförmigen Querschnitt aufweisen. Solche Führungselemente sind Fachleuten bekannt. Die Führungselemente können aus Metall oder anderen Materialien ausgebildet sein. Besonders bevorzugt sind die Führungselemente Führungsbleche. Zwischen dem Elektrodeninstrument, bzw. dessen Führungselementen, und der Innenwandung des Innenrohres sind in der Regel keine weiteren Teile angeordnet. Innerhalb des Innenrohres können jedoch weitere Komponenten, wie z.B. eine Optik angeordnet sein.

Zusätzlich oder alternativ zu diesen Führungselementen weist, wie oben beschrieben, der erfindungsgemäße Isoliereinsatz Verbindungselemente auf, mittels derer das Elektrodeninstrument radial gegen eine Verschiebung gesichert ist, während es axial beweglich bleibt. Es ist im Rahmen der Erfindung möglich, auf die üblichen, oben beschriebenen Führungselemente zu verzichten, da das Elektrodeninstrument an dem Isoliereinsatz geführt wird. Es versteht sich aber, dass eine zusätzliche Stabilisierung durch Führungselemente auch im Rahmen dieser Erfindung angedacht ist.

Der Resektoskopschaft umfasst in der Regel Elemente zur Herstellung der an anderer Stelle beschriebenen lösbaren Verbindung mit dem Isoliereinsatz, insbesondere zur lösbaren Verbindung des distalen Endbereichs des Hüllrohres oder des Innenrohres mit dem proximalen Endbereich des Isoliereinsatzes. Diese Elemente sind jeweils funktionskomplementär zu den oben beschriebenen Befestigungsmitteln des Isoliereinsatzes, sodass die Elemente mit den Befestigungsmitteln eine lösbare Verbindung zwischen Isoliereinsatz und Resektoskopschaft ausbilden. Das Hüllrohr bzw. das Innenrohr des Resektoskops kann beispielsweise zur Ausbildung der lösbaren Verbindung eine Eingriffsöffnung für ein Rastelement des Isoliereinsatzes aufweist. Alternativ oder zusätzlich kann das Hüllrohr oder das Innenrohr eine oder mehrere radiale Protrusionen umfassen, die im montierten Zustand eine axiale Verschiebung des Isoliereinsatzes in distale Richtung verhindern, zum Beispiel eines Isoliereinsatzes der eine konische Form aufweist.

### Kurze Beschreibung der Figuren

In den Zeichnungen sind Ausführungsbeispiele der Erfindung schematisch dargestellt. Es zeigen:
- **Fig. 1**: eine schematische, seitliche Schnittdarstellung eines Resektoskops aus dem Stand der Technik in dem am Innenrohr eine Isolierspitze angeordnet ist;
- **Fig. 2**: eine schematische, seitliche Schnittdarstellung eines alternativen Resektoskops aus dem Stand der Technik in dem am Hüllrohr eine Isolierspitze angeordnet ist;
- **Fig. 3**: eine schematische, seitliche Schnittdarstellung des distalen Endbereichs des in Fig. 1 gezeigten Resektoskops aus dem Stand der Technik;
- **Fig. 4**: eine schematische, seitliche Schnittdarstellung des distalen Endbereichs des in Fig. 2 gezeigten Resektoskops aus dem Stand der Technik;
- **Fig. 5**: eine schematische, seitliche Schnittdarstellung des distalen Endbereichs eines erfindungsgemäßen Resektoskops, das einen Isoliereinsatz mit radial außen angeordneten Befestigungsmitteln aufweist;
- **Fig. 6**: eine schematische, seitliche Schnittdarstellung des distalen Endbereichs eines alternativen erfindungsgemäßen Resektoskops, das einen Isoliereinsatz mit einer konischen Form in dessen proximalen Endbereich aufweist;
- **Fig. 7**: eine schematische, seitliche Schnittdarstellung des distalen Endbereichs eines alternativen erfindungsgemäßen Resektoskops, das einen Isoliereinsatz mit einer konischen Form in dessen proximalen Endbereich aufweist und in dem ein Elektrodeninstrument mit dem Isoliereinsatz axial verschiebbar verbunden ist;
- **Fig. 8**: eine schematische, seitliche Schnittdarstellung des distalen Endbereichs eines alternativen erfindungsgemäßen Resektoskops, das einen Isoliereinsatz mit einer konischen Form in dessen proximalen Endbereich aufweist, in dem ein Elektrodeninstrument mit dem Isoliereinsatz axial verschiebbar verbunden ist und dessen Isoliereinsatz ein Rastelement zur zusätzlichen Verbindung zwischen Isoliereinsatz und Resektoskopschaft aufweist (A) und eine Vergrößerung eines Ausschnitts dieser Schnittdarstellung (B); und
- **Fig. 9**: einen Ausschnitt einer schematischen, seitlichen Schnittdarstellung des distalen Endbereichs eines weiteren erfindungsgemäßen Resektoskops, dessen Isoliereinsatz ein Rastelement zur zusätzlichen Verbindung zwischen Isoliereinsatz und Resektoskopschaft aufweist, wobei die Rastverbindung ohne Zerstören des Rastelements gelöst werden kann.

### Ausführungsbeispiele

Weitere Vorteile, Kennzeichen und Merkmale der vorliegenden Erfindung werden bei der nachfolgenden detaillierten Beschreibung von Ausführungsbeispielen anhand der beigefügten Zeichnungen deutlich. Allerdings ist die Erfindung nicht auf diese Ausführungsbeispiele beschränkt.

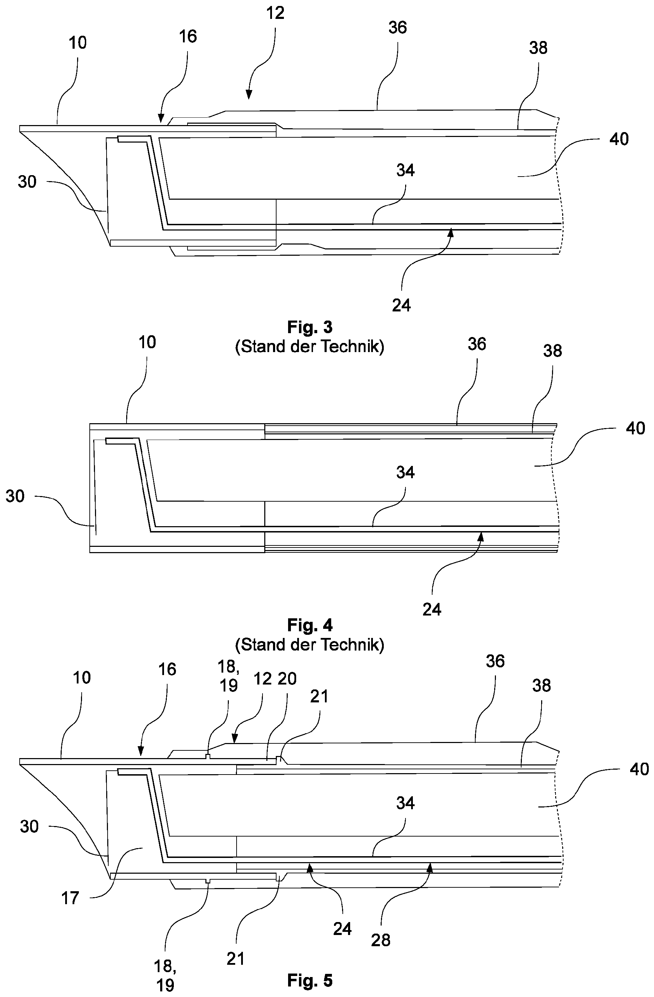

Fig. 1 zeigt eine schematische, seitliche Schnittdarstellung eines Resektoskops 26 aus dem Stand der Technik in dem am Innenrohr 38 ein Isoliereinsatz 10 angeordnet ist. Fig. 3 zeigt eine schematische, seitliche Schnittdarstellung des distalen Endbereichs desselben Resektoskops aus dem Stand der Technik.

Das Resektoskop 26 weist einen Resektoskopschaft 14 auf, der ein gestrichelt dargestelltes Hüllrohr 36 (Außenrohr) umfasst. Innerhalb des Hüllrohres 36 verläuft ein Innenrohr 38 und innerhalb des Innenrohres 38 ein Elektrodeninstrument 24 sowie eine in Fig. 3 dargestellte Optik 40 und ein Beleuchtungsmittel, zum Beispiel in Form eines Lichtleitfaserbündels. Darüber hinaus können weitere hier nicht dargestellte Elemente in den Resektoskopen verlaufen, wie beispielsweise ein separates Spülrohr und dergleichen. Das Hüllrohr 36 umfasst in seinem distalen Endbereich in hier nicht dargestellter Weise Öffnungen durch die verunreinigte Spülflüssigkeit in den Zwischenraum zwischen Hüllrohr 36 und Innenrohr 38 einströmen und durch den Resektoskopschaft 14 abfließen kann.

Wie in Fig. 1 und in größerem Detail in der Fig. 3 erkennbar ist, ist das Elektrodeninstrument 24 in diesem üblichen Instrument mittels eines Halteelements 46 (Führungselement) mit einem teilkreisförmigen Querschnitt gegen Querverschiebungen, d.h. Verschiebungen abweichend von der Längsrichtung des Resektoskopschaftes 14, zum Beispiel quer zur Längsrichtung, geschützt. Das Elektrodeninstrument 24 ist längsverschiebbar in dem Innenrohr 38 gelagert. Das Halteelement 46 ist formkomplementär zur Innenwandung des Innenrohres 38 oder zur Außenwand der Optik 40 ausgebildet und weist eine teilzylindrische Form auf. Das Halteelement 46 ist in einem Schaftabschnitt des Elektrodeninstruments 24 an zwei Gabelrohren 34 befestigt. Die Gabelrohre 34 verlaufen innerhalb des Resektoskopschaftes 14 dicht nebeneinander und laufen erst im distalen Endbereich des Resektoskopschaftes 14 auseinander, um zwischen ihren Enden eine Schlingenelektrode aufzunehmen und zu tragen. Alternativ ist es auch denkbar, dass die Gabelrohre 34 im mittleren oder proximalen Bereich des Resektoskops 26 in ein Elektrodeninstrument-Schaftrohr übergehen. In dieser Ausführungsform können das oder die Halteelemente 46 an dem Elektrodeninstrument-Schaftrohr angeordnet sein.

Das Elektrodeninstrument 24 kann durch Betätigung eines Handgriffs 48 zwangsgeführt axial in distale und proximale Richtung bewegt werden. Dabei kann es über das distale Ende des Innenrohres 38 und des Hüllrohres 36 hinausgeschoben werden. So wird es dem Operateur ermöglicht, auch weiter von der Resektoskopspitze entferntes Gewebe zu manipulieren. Zu diesem Zweck sind ferner Innenrohr 38 und/oder das Elektrodeninstrument 24 um ihre Längsachsen drehbar gelagert. Das Elektrodeninstrument 24 weist an seinem distalen Ende eine Elektrode 30 auf, die als Schneidschlinge ausgebildet ist und mittels derer Gewebe durch elektrochirurgische Ablation entfernt werden kann. Hierbei wird an die Elektrode 30 eine hochfrequente, elektrische Spannung angelegt, um Gewebe zu zerschneiden.

Das dargestellte Resektoskop 26 weist einen passiven Transporteur auf, bei dem der Schlitten 56 durch Relativbewegung der proximal von dem Resekoskopschaft 14 angeordneten Griffteile 50 und 52 zueinander gegen eine von einer Federbrücke 54 aufgebrachte Federkraft in distale Richtung gegen das distale, erste Griffteil 52 verschoben wird. Bei der Verschiebung des Schlittens 56 in distale Richtung gegen das Griffteil 52 wird das Elektrodeninstrument 24 in nicht dargestellter Weise zwangsgeführt nach distal verschoben. Bei einer Entlastung der Handgriffteile 50, 52 zwingt die von der Federbrücke 54 erzeugte Federkraft den Schlitten 56 zurück in seine Ruheposition, wobei das Elektrodeninstrument 24 in proximale Richtung gezogen wird. Bei der Rückverschiebung des Schlittens 56 kann ohne Handkraft des Operateurs, also passiv, ein elektrochirurgischer Eingriff mit dem Elektrodeninstrument 24 vorgenommen werden.

Im Gegensatz zu dem erfindungsgemäßen Isoliereinsatz 10, ist der Isoliereinsatz 10 dieses üblichen Resektoskops 26 nicht lösbar mit dem Resektoskopschaft 14 verbunden, sondern durch eine hier nicht dargestellte Verklebung permanent an dem Resektoskopschaft 14 fixiert. Darüber hinaus umfasst der Isoliereinsatz 10 auch keine Befestigungsmittel 18.

Fig. 2 zeigt eine schematische, seitliche Schnittdarstellung eines alternativen Resektoskops 26 aus dem Stand der Technik in dem der Isoliereinsatz 10 am Hüllrohr 36 angeordnet ist. Fig. 4 zeigt eine schematische, seitliche Schnittdarstellung des distalen Endbereichs dieses bekannten Resektoskops 26. Durch die Anbringung des Isoliereinsatzes 10 am Hüllrohr 36 ist es möglich, den Resektoskopschaft 14 in einer ultradünnen Bauweise auszubilden. Das Resektoskop 26 entspricht darüber hinaus im Wesentlichen dem in den Fig. 1 und 3 dargestellten Resektoskop 26. Auch in dem in Fig. 2 und 4 dargestellten Resektoskop 26 sind Isoliereinsatz 10 und Resektoskopschaft 14 durch eine permanente Verklebung miteinander verbunden.

Im Gegensatz zu dieser üblichen permanenten Verbindung zwischen Isoliereinsatz 10 und Resektoskopschaft 14 zeigen die Fig. 5 bis 8 schematische, seitliche Schnittdarstellungen des distalen Endbereichs 12 verschiedener erfindungsgemäßer Resektoskope 26 in denen die Verbindung zwischen Isoliereinsatz 10 und Resektoskopschaft 14 lösbar ausgebildet ist. Abgesehen von den untenstehend beschriebenen, erfindungsgemäßen Unterschieden gleichen die dargestellten Resektoskope 26 im Wesentlichen den in den Fig. 1 bis 4 dargestellten Resektoskopen 26.

Fig. 5 zeigt eine Ausführungsform, die einen Isoliereinsatz mit radial außen angeordneten Befestigungsmitteln 18 aufweist. Die Befestigungsmittel 18 sind als zapfenförmige Extrusionen auf der Außenseite der Wand 20 des Isoliereinsatzes ausgebildet, d.h. als Verdickungen 19 der Wand 20. Der Isoliereinsatz 10 weist hier mehr als ein Befestigungsmittel 18 auf, nämlich mindestens zwei Befestigungsmittel 18, vorzugsweise 3, die noppenartig gleichmäßig entlang eines Umfangs des Isoliereinsatzes 10 angeordnet sind. Es ist ebenfalls denkbar, stattdessen ein einziges Befestigungsmittel als umlaufenden Wulst an dem Isoliereinsatz 10 anzuordnen. Das Innenrohr 38 weist in der hier gezeigten Ausführungsform zwei oder mehr Anlageelemente 21 mit einer distalen Anlagefläche auf, die mit dem proximalen Ende des Isoliereinsatzes 10 in Anschlag bringbar sind und auf diese Weise ein Verschieben des Isoliereinsatzes 10 in proximale Richtung verhindern.

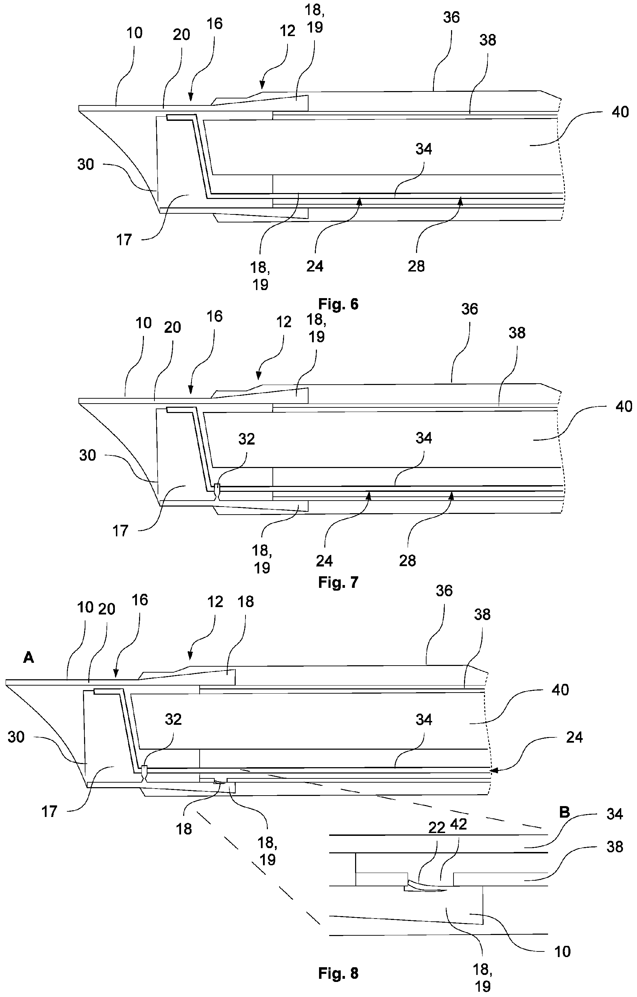

Fig. 6 zeigt einen Isoliereinsatz 10, dessen Wand 20 an ihrer Außenseite eine konische Form im proximalen Endbereich des Isoliereinsatzes 10 aufweist. Mit anderen Worten ist der Durchmesser des Isoliereinsatzes 10 an seinem proximalen Ende größer als an dem distalen Ende seines hohlen Abschnitts 16. Es ist erkennbar, dass der Isoliereinsatz 10 zwischen dem Hüllrohr 38 und dem Innenrohr 36 angeordnet ist und ein Verrutschen des konisch geformten Isoliereinsatzes 10 in distale Richtung durch eine Verengung des Hüllrohres 36 an dessen distalen Ende verhindert wird.

Fig. 7 zeigt eine Ausführungsform in der das Resektoskop 26 aus Fig. 6 durch eine Verbindung zwischen Isoliereinsatz 10 und Elektrodeninstrument 24 ergänzt ist. Das Elektrodeninstrument 24 ist mittels eines oder mehrerer Verbindungselemente 32 mit dem Isoliereinsatz 10 axial verschiebbar verbunden. Jedes der vorzugsweise zwei Verbindungselemente 32 ist hohlzylinderförmig, wobei die Längsachse des Hohlzylinders sich parallel zu der Längsachse des Resektoskopschaftes 14 erstreckt und im Inneren des Hohlzylinders jeweils ein Gabelrohr 34 des Elektrodeninstruments 24 axial verschiebbar geführt wird.

Fig. 8 zeigt eine Ausführungsform in dem das Resektoskop 26 aus Fig. 7 durch eine zusätzliche, lösbare Verbindung zwischen Isoliereinsatz 10 und Resektoskopschaft 14 ergänzt ist. Es versteht sich, dass es im Rahmen der Erfindung auch möglich ist, zugunsten dieser weiteren lösbaren Verbindung auf die konische Form des Isoliereinsatzes 10 und/oder auf das Verbindungselement 32 zu verzichten. Der Isoliereinsatz 10 weist hier zur Herstellung einer weiteren lösbaren Verbindung ein Rastelement 22 auf. Wie in der im Abbildungsteil B gezeigten Vergrößerung erkennbar ist, ist das Rastelement 22 in diesem Falle ein länglicher, mit der Innenwand des Isoliereinsatzes 10 verbundener Teilausschnitt der Innenwand des Isoliereinsatzes 10. Der Teilausschnitt ist in flachem Winkel von der Innenwand nach distal-innen abgewinkelt. Die angrenzende Außenwand des Innenrohrs 38 weist eine entsprechende Eingriffsöffnung 42 auf. Wird der Isoliereinsatz 10 in die dargestellte Stellung über den distalen Endbereich des Innenrohrs 38 geschoben, so gelangt der Teilausschnitt in Eingriff mit der Eingriffsöffnung 42. Durch Ausübung einer entsprechenden Zugkraft kann der Isoliereinsatz 10 wieder gelöst und der Teilausschnitt dabei abgebrochen werden.

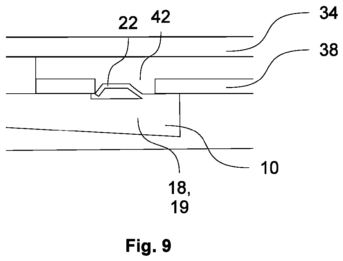

Fig. 9 zeigt, wie Fig. 8 B, einen vergrößerten Ausschnitt eines erfindungsgemäßen Resektoskops. Der Isoliereinsatz 10 weist zur Herstellung einer lösbaren Verbindung ein Rastelement 22 auf. Das Rastelement 22 ist ein länglicher, mit der Innenwand des Isoliereinsatzes 10 verbundener Teilausschnitt der Innenwand des Isoliereinsatzes 10. Der Teilausschnitt umfasst einen proximalen, im flachen Winkel von der Innenwand nach distal-innen abgewinkelten Abschnitt, einen distal davon angeordneten Abschnitt, der im Wesentlichen parallel zur Längsachse des Resektoskops 26 verläuft, und einen distalen Abschnitt, der nach distal-außen abgewinkelt ist. Die angrenzende Außenwand des Innenrohrs 38 weist eine entsprechende Eingriffsöffnung 42 auf. Wird der Isoliereinsatz 10 in die dargestellte Stellung über den distalen Endbereich des Innenrohrs 38 geschoben, so gelangt der Teilausschnitt in Eingriff mit der Eingriffsöffnung 42. Durch Ausübung einer entsprechenden Zugkraft kann der Isoliereinsatz 10 wieder gelöst werden, ohne den Teilausschnitt abzubrechen. Dies wird insbesondere dadurch sichergestellt, dass das Rastelement 22 in seinem distalen Endbereich eine schräg nach proximal-innen verlaufende Fläche aufweist.

Obwohl die vorliegende Erfindung anhand der Ausführungsbeispiele detailliert beschrieben worden ist, ist für den Fachmann selbstverständlich, dass die Erfindung nicht auf diese Ausführungsbeispiele beschränkt ist, sondern dass vielmehr Abwandlungen in der Weise möglich sind, dass einzelne Merkmale weggelassen oder andersartige Kombinationen der vorgestellten Einzelmerkmale verwirklicht werden können, sofern der Schutzbereich der beigefügten Ansprüche nicht verlassen wird.

**Bezugszeichenliste**

| | | | |
|---|---|---|---|
| 10 | Isoliereinsatz | 48 | Handgriff |
| 12 | Endbereich | 50 | Griffteil |
| 14 | Resektoskopschaft | 52 | Griffteil |
| 16 | hohler Abschnitt | 54 | Federbrücke |
| 17 | Hohlraum | 56 | Schlitten |
| 18 | Befestigungsmittel | | |
| 19 | Verdickung | | |
| 20 | Wand | | |
| 21 | Anlageelemente | | |
| 22 | Rastelement | | |
| 24 | Elektrodeninstrument | | |
| 26 | Resektoskop | | |
| 28 | Schaftabschnitt | | |
| 30 | Elektrode | | |
| 32 | Verbindungselement | | |
| 34 | Gabelrohr | | |
| 36 | Hüllrohr | | |
| 38 | Innenrohr | | |
| 40 | Optik | | |
| 42 | Eingriffsöffnung | | |
| 46 | Halteelement | | |

## Patentansprüche

1. Elektrisch isolierender Isoliereinsatz (10) zur lösbaren Verbindung mit dem distalen Endbereich (12) eines Resektoskopschaftes (14), wobei der Isoliereinsatz (10) einen hohlen Abschnitt (16) mit einem länglichen Hohlraum (17) zum Hindurchführen von Durchgangsinstrumenten und Befestigungsmittel (18) zur lösbaren Verbindung mit dem Resektoskopschaft (14) aufweist, wobei das Befestigungsmittel (18) eine radiale Verdickung (19) des Isoliereinsatzes (10) in dessen hohlen Abschnitt (16) umfasst oder aus dieser besteht und das Befestigungsmittel (18) einen Teil einer Bajonett-, Schraub-, Schnapp-, Klemm- oder Rastverbindung umfasst oder aus diesem besteht, wobei die radiale Verdickung (19) in einem größeren Außendurchmesser des Isoliereinsatzes (10) im Verhältnis zu dem Außendurchmesser am distalen Ende des hohlen Abschnitts (16) und
in einem größeren Durchmesser der Wand (20) des Isoliereinsatzes (10) im Verhältnis zu dem Durchmesser der Wand (20) am distalen Ende des hohlen Abschnitts (16) besteht,
**dadurch gekennzeichnet, dass**
auf einer Innenwand des Isoliereinsatzes (10) mehrere Protrusionen ausgebildet sind, die radial entlang eines Umfangs auf der Innenwand umlaufend ausgebildet sind.

2. Isoliereinsatz (10) nach Anspruch 1, **dadurch gekennzeichnet, dass** das Befestigungsmittel (18) ein an der Befestigungsseite des hohlen Abschnitts (16) angeordnetes, vorstehendes Rastelement (22) umfasst.

3. Isoliereinsatz (10) nach einem der vorangegangenen Ansprüche, **dadurch gekennzeichnet, dass** die Befestigungsseite des hohlen Abschnitts (16) dessen Außenwand ist.

4. Isoliereinsatz (10) nach einem der vorangegangenen Ansprüche, **dadurch gekennzeichnet, dass** der Isoliereinsatz (10) aus Kunststoff ist.

5. Isoliereinsatz (10) nach Anspruch 4, **dadurch gekennzeichnet, dass** der Kunststoff ein thermostabiler Kunststoff ist.

6. Elektrodeninstrument (24) zur Verwendung in einem Resektoskop (26), wobei das Elektrodeninstrument (24) einen Schaftabschnitt (28) und an seinem distalen Ende eine mit Hochfrequenzstrom beaufschlagbare Elektrode (30) aufweist, **dadurch gekennzeichnet, dass** das Elektrodeninstrument (24) mit einem Isoliereinsatz (10) gemäß einem der vorangehenden Ansprüche verbunden ist und das Elektrodeninstrument (24) und der Isoliereinsatz (10) im Verhältnis zueinander axial verschiebbar sind.

7. Elektrodeninstrument (24) nach Anspruch 6, **dadurch gekennzeichnet, dass** der Isoliereinsatz (10) ein oder mehr, vorzugsweise zwei Verbindungselemente (32) aufweist, in denen jeweils ein Gabelrohr (34) des Elektrodeninstruments (24) axial verschiebbar gelagert ist.

8. Resektoskop (26) für die endoskopische Chirurgie mit einem rohrartigen Resektoskopschaft (14), der ein langgestrecktes Hüllrohr (36) und ein in dem Hüllrohr angeordnetes Innenrohr (38) sowie eine stabförmige Optik (40) umfasst, **dadurch gekennzeichnet, dass** das Resektoskop (26)
a) einen Isoliereinsatz (10) nach einem der Ansprüche 1 bis 5 sowie ein Elektrodeninstrument (24) mit einem Schaftabschnitt (28) und mit einer mit Hochfrequenzstrom beaufschlagbaren Elektrode (30) an seinem distalen Ende aufweist; oder
b) ein Elektrodeninstrument (24) nach einem der Ansprüche 6 bis 7 aufweist.

9. Resektoskop (26) nach Anspruch 8, **dadurch gekennzeichnet, dass** die Wand (20) des Isoliereinsatzes (10) in dessen proximalen Endbereich zwischen dem Hüllrohr (36) und dem innerhalb des Hüllrohres verlaufenden Innenrohr (38) angeordnet ist.

10. Resektoskop (26) nach einem der Ansprüche 8 bis 9, **dadurch gekennzeichnet, dass** der Isoliereinsatz (10) mit dem distalen Endbereich des Hüllrohres (36) oder des Innenrohres (38) lösbar verbunden ist und das Hüllrohr (36) bzw. das Innenrohr (38) zur Ausbildung der lösbaren Verbindung eine Eingriffsöffnung (42) für ein Rastelement (22) des Isoliereinsatzes (10) aufweist.

## Claims

1. An electrically insulating insert (10) for detachable connection to the distal end region (12) of a resectoscope shaft (14), wherein the insulating insert (10) has a hollow portion (16) with an elongate cavity (17) for the passage of pass-through instruments and fastening means (18) for detachable connection to the resectoscope shaft (14), wherein the fastening means (18) comprises or consists of a radial thickening (19) of the insulating insert (10) in the hollow portion (16) thereof and the fastening means (18) comprise or consist of a part of a bayonet, screw, snap, clamp, or locking connection, wherein the radial thickening (19) consists in a larger outer diameter of the insulating insert (10) relative to the outer diameter at the distal end of the hollow portion (16), and in a larger diameter of the wall (20) of the insulating insert (10) relative to the diameter of the wall (20) at the distal end of the hollow portion (16), **characterized in that** a plurality of protrusions are formed on an inner wall of the insulating insert (10), said protrusions being formed so as to run radially along a circumference on the inner wall.

2. The insulating insert (10) claimed in claim 1, **characterized in that** the fastening means (18) comprises a protruding locking element (22) that is arranged on the fastening side of the hollow portion (16).

3. The insulating insert (10) as claimed in any one of the preceding claims, **characterized in that** the fastening side of the hollow portion (16) is the outer wall thereof.

4. The insulating insert (10) as claimed in any one of the preceding claims, **characterized in that** the insulating insert (10) is made of plastic.

5. The insulating insert (10) as set forth in claim 4, **characterized in that** the plastic is a thermostable plastic.

6. An electrode instrument (24) for use in a resectoscope (26), the electrode instrument (24) having a shaft portion (28) and, at its distal end, an electrode (30) to which high-frequency current can be applied, **characterized in that** the electrode instrument (24) is connected to an insulating insert (10) as claimed in any one of the preceding claims and the electrode instrument (24) and the insulating insert (10) can be displaced axially relative to one another.

7. The electrode instrument (24) as claimed in claim 6, **characterized in that** the insulating insert (10) has one or more, preferably two connecting elements (32), in each of which a fork tube (34) of the electrode instrument (24) is supported in an axially displaceable manner.

8. A resectoscope (26) for endoscopic surgery with a tubular resectoscope shaft (14) that comprises an elongate cladding tube (36) and an inner tube (38) that is arranged in the cladding tube, as well as rod-shaped optics (40), **characterized in that** the resectoscope (26) has
a) an insulating insert (10) as claimed in any one of claims 1 to 5 and an electrode instrument (24) with a shaft portion (28) and with an electrode (30) to which high-frequency current can be applied at its distal end; or
b) an electrode instrument (24) as claimed in any one of claims 6 to 7.

9. The resectoscope (26) as claimed in claim 8, **characterized in that** the wall (20) of the insulating insert (10) is arranged in its proximal end region between the cladding tube (36) and the inner tube (38) running inside the cladding tube.

10. The resectoscope (26) as claimed in any one of claims 8 to 9, **characterized in that** the insulating insert (10) is detachably connected to the distal end region of the cladding tube (36) or of the inner tube (38), and that the cladding tube (36) or the inner tube (38) has an engagement opening (42) for a locking element (22) of the insulating insert (10) in order to form the detachable connection.

## Revendications

1. Insert isolant électriquement (10) destiné à une liaison amovible avec la zone d'extrémité distale (12) d'un arbre de résectoscope (14), l'insert isolant (10) présentant une section creuse (16) avec une cavité allongée (17) pour le passage d'instruments traversants et des moyens de fixation (18) pour la liaison amovible avec l'arbre de résectoscope (14), les moyens de fixation (18) comprenant ou consistant en un épaississement radial (19) de l'insert isolant (10) dans sa section creuse (16), et les moyens de fixation (18) comprenant ou consistant en une partie d'une liaison à baïonnette, à vis, à encliquetage, à serrage ou à cliquet, l'épaississement radial (19) consistant en un diamètre extérieur plus grand de l'insert isolant (10) par rapport au diamètre extérieur à l'extrémité distale de la section creuse (16) et
en un diamètre plus grand de la paroi (20) de l'insert isolant (10) par rapport au diamètre de la paroi (20) à l'extrémité distale de la section creuse (16), **caractérisé en ce que** plusieurs protubérances sont formées sur une paroi intérieure de l'insert isolant (10), lesquelles sont formées radialement de manière circonférentielle le long d'une périphérie sur la paroi intérieure.

2. Insert isolant (10) selon la revendication 1, **caractérisé en ce que** le moyen de fixation (18) comprend un élément d'encliquetage saillant (22) disposé sur le côté de fixation de la section creuse (16).

3. Insert isolant (10) selon l'une des revendications précédentes, **caractérisé en ce que** le côté de fixation de la section creuse (16) est sa paroi extérieure.

4. Insert isolant (10) selon l'une des revendications précédentes, **caractérisé en ce que** l'insert isolant (10) est en matière plastique.

5. Insert isolant (10) selon la revendication 4, **caractérisé en ce que** la matière plastique est une matière plastique thermostable.

6. Instrument à électrode (24) destiné à être utilisé dans un résectoscope (26), l'instrument à électrode (24) comprenant une section d'arbre (28) et, à son extrémité distale, une électrode (30) pouvant être alimentée en courant haute fréquence, **caractérisé en ce que** l'instrument à électrode (24) est relié à un insert isolant (10) selon l'une des revendications précédentes et **en ce que** l'instrument à électrode (24) et l'insert isolant (10) peuvent être déplacés axialement l'un par rapport à l'autre.

7. Instrument à électrode (24) selon la revendication 6, **caractérisé en ce que** l'insert isolant (10) présente un ou plusieurs, de préférence deux, éléments de liaison (32), dans lesquels un tube en fourche (34) de l'instrument à électrode (24) est, dans chaque cas, monté de manière axialement déplaçable.

8. Résectoscope (26) pour la chirurgie endoscopique comprenant un arbre de résectoscope tubulaire (14), lequel comprend un tube d'enveloppe allongé (36) et un tube intérieur (38) disposé dans le tube extérieur, ainsi qu'une optique en forme de tige (40), **caractérisé en ce que** le résectoscope (26)
a) comprend un insert isolant (10) selon l'une des revendications 1 à 5 ainsi qu'un instrument à électrode (24) comprenant une section d'arbre (28) et une électrode (30) pouvant être alimentée en courant haute fréquence à son extrémité distale ; ou
b) comprend un instrument à électrode (24) selon l'une des revendications 6 à 7.

9. Résectoscope (26) selon la revendication 8, **caractérisé en ce que** la paroi (20) de l'insert isolant (10) est disposée, dans sa zone d'extrémité proximale, entre le tube d'enveloppe (36) et le tube intérieur (38) s'étendant à l'intérieur du tube d'enveloppe.

10. Résectoscope (26) selon l'une des revendications 8 à 9, **caractérisé en ce que** l'insert isolant (10) est relié de manière amovible à la zone d'extrémité distale du tube d'enveloppe (36) ou du tube intérieur (38), et **en ce que** le tube d'enveloppe (36) ou le tube intérieur (38) présente, pour la réalisation de la liaison amovible, une ouverture d'engagement (42) pour un élément d'encliquetage (22) de l'insert isolant (10).
